# FASCICULE DE BREVET EUROPEEN

(11) **EP 0 495 907 B1**
(45) Date de publication et mention de la délivrance du brevet: **29.07.1998**
(21) Numéro de dépôt: 90916311.5
(22) Date de dépôt: 19.10.1990
(51) Int. Cl.: C12N 15/12, C07K 14/56, C12N 5/10, C12P 21/08, G01N 33/566, G01N 33/577, G01N 33/68

(54) **FRAGMENT D'ADNc CODANT POUR LE GENE DU RECEPTEUR DE L'INTERFERON ALPHA ET PROCEDE DE PREPARATION D'UNE PROTEINE CORRESPONDANTE**
cDNS-FRAGMENT, DAS FÜR EIN INTERFERON-ALPHA-REZEPTOR-GEN KODIERT UND VERFAHREN ZUR HERSTELLUNG EINES ENTSPRECHENDEN PROTEINS
cDNA FRAGMENT CODING THE ALPHA INTERFERON RECEPTOR GENE AND PROCESS FOR THE PREPARATION OF A CORRESPONDING PROTEIN

(30) Priorité: 20.10.1989 FR 8913770
(43) Date de publication de la demande: 29.07.1992
(73) Titulaire: LABORATOIRE EUROPEEN DE BIOTECHNOLOGIE S.A., 75008 Paris (FR)
(72) Inventeur: MOGENSEN, Knud, Erik, F-75013 Paris (FR); UZE, Gilles, F-75011 Paris (FR); LUTFALLA, Georges, F-75007 Paris (FR); GRESSER, Ion, F-75007 Paris (FR)
(74) Mandataire: Desaix, Anne
(86) Numéro de dépôt international: FR9000758
(87) Numéro de publication internationale: WO9105862

(56) Documents cités:
- Journal Interferon Research, volume 8, suppl. 1, 1988, New York, US), L. Shulman et Al.: "Molecular cloning of the human IFN-alpha, beta receptor cDNA", page S16, résumé no. 3-9
- ISCU Short Report, volume 4, 1986, M. Revel et al.: "Interferon receptor and interferon-activated genes", pages 362-365
- Cell, volume 60, 26 janvier 1990, Cell Press, (Cambridge, GB), G. Uzé et al.: "Genetic transfer of a functional human interferon receptor into mouse cells: cloning and expression of its cDNA", pages 225-234
- Biochemical and Biophysical Research Communications, volume 107, no. 3, 16 aout 1982, Academic Press, Inc., (New York, US), C.J. Epstein et al.: "Direct evidence that the gene product of the human chromosome 21 locus, IFRC, is the interferon-alpha receptor", pages 1060-1066
- Proceedings National Academy Science, volume 81, Septembre 1984, (US), A. Raziuddin et al.: "Receptors for human alpha and beta interferon but not for gamma interferon are specified by human chromosome 21", pages 5504-5508

## Description

La présente invention se rapporte à la séquence, notamment à une séquence d'ADNc codant pour le gène du récepteur de l'interféron alpha.

L'interféron est un terme générique désignant trois classes antigéniques : alpha, béta et gamma, de protéines capables, entre autres, d'induire un état antiviral et d'inhiber la multiplication des cellules sensibles.

Il existe entre les interférons alpha et béta, qui sont produits à la suite d'infection virale, une homologie de structure suffisamment forte pour que ces deux types d'interféron puissent réagir par voie du même récepteur cellulaire.

L'interféron alpha humain est lui-même un mélange d'une douzaine de protéines à fort pourcentage d'homologie, codées par des gènes de structure différente. Ces sous-types ont un spectre de fonction identique mais leurs activités spécifiques sont différentes.

L'interféron gamma, qui est produit par les lymphocytes activés, ne possède aucune homologie avec les interférons alpha/béta et il ne réagit pas avec leur récepteur.

Actuellement, les structures des interférons (qui possèdent environ 165 acides aminés) sont assez bien connues au niveau de leur séquence d'acides aminés et plusieurs études ont été dirigées vers l'analyse des domaines fonctionnels de ces protéines. Des molécules hybrides, construites entre les interférons de haute et de basse affinité en utilisant des sites de restriction de l'ADN codant pour ces interférons, ont été utilisées pour montrer que la partie N-terminale de la molécule déterminait l'affinité de liaison avec son récepteur cellulaire et par conséquence, l'activité spécifique des interférons alpha.

Les maladies respiratoires d'origines virales posent d'importants problèmes économiques pour la santé publique. Les essais cliniques ont montré que l'interféron alpha protège à 100 % des volontaires infectés par différents rhinovirus. De même, il a été mis en évidence que parmi les volontaires traités à l'interféron alpha et infectés par un coronavirus. 6 % ont développé les symptômes du rhume comparé à 37 % des volontaires traités par placebo.

Néanmoins, quoique l'interféron alpha humain se montre efficace dans ces essais, la toxicité qu'il exerce sur la muqueuse nasale pose un problème majeur.

L'interféron possède également une activité antitumorale chez l'homme et à l'heure actuelle il est devenu le traitement de choix pour certains cancers. Cependant, l'interféron injecté par voie générale exerce également une toxicité pour le système nerveux ce qui limite les possibilités de traitement.

En fait, actuellement, il n'y a aucun moyen pour déterminer quel devrait être l'interféron à utiliser pour obtenir une activité thérapeutique et une toxicité réduite. Plusieurs laboratoires ont déployé des efforts considérables pour construire des interférons modifiés qui auraient une activité prononcée accompagnée d'une faible toxicité. Cette approche s'est révélée décevante.

Il est maintenant évident que le succès éventuel d'un tel projet nécessite la connaissance de la structure du récepteur des interférons pour imaginer la construction de l'agoniste. Un agoniste ayant une activité élevée et une faible toxicité pour la muqueuse nasale trouverait un marché très important pour le traitement des maladies respiratoires d'origines virales.

La publication de Shulman L. et al (Molecular cloning of the human IFN-α,β réceptor cDNA - Journal Interferon Research, vol. 8, suppl. 1, 1988, New York, US p. 516 résumé n° 3-9) et la publication de Revel M. et al (Interferon receptor and interferon-activated genes, ISCU Short Report, vol. 4, 1986, p. 362-365) décrivent le transfert de fragments d'ADN humain dans des cellules et l'obtention d'une réponse de certaines des cellules ainsi transformées à l'interféron humain a et/ou à l'interféron humain β.

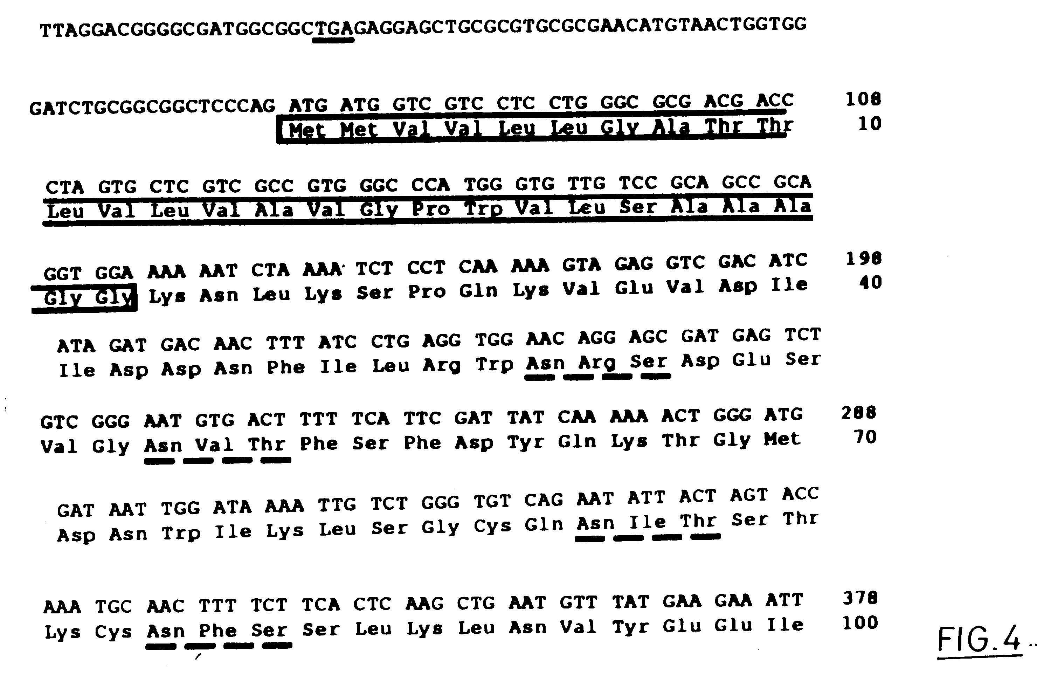

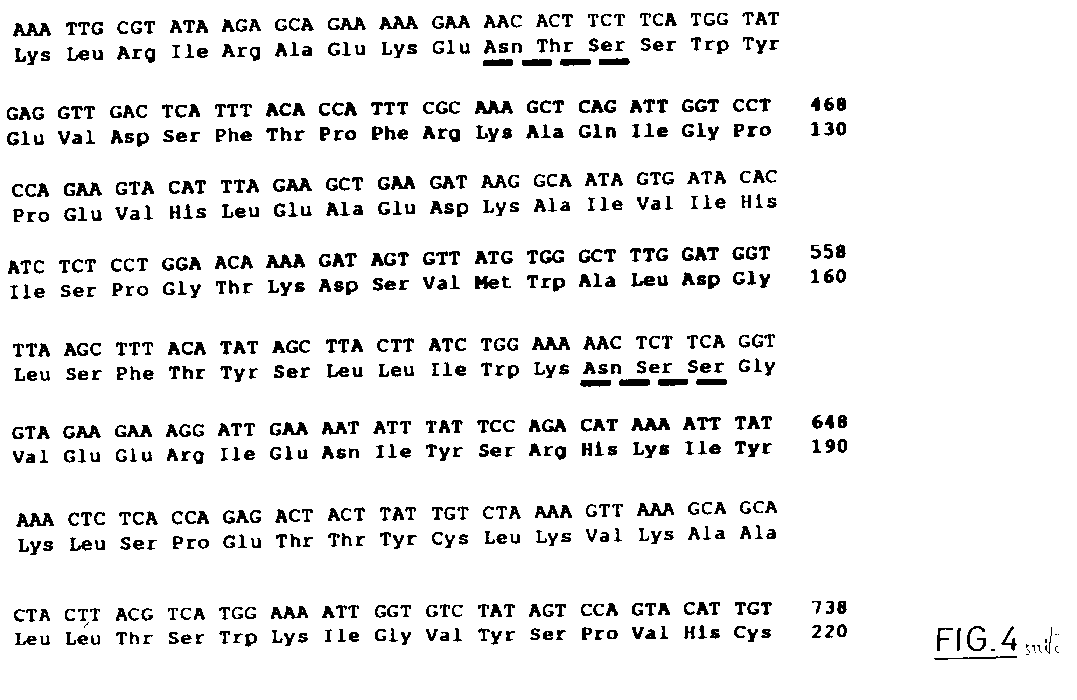

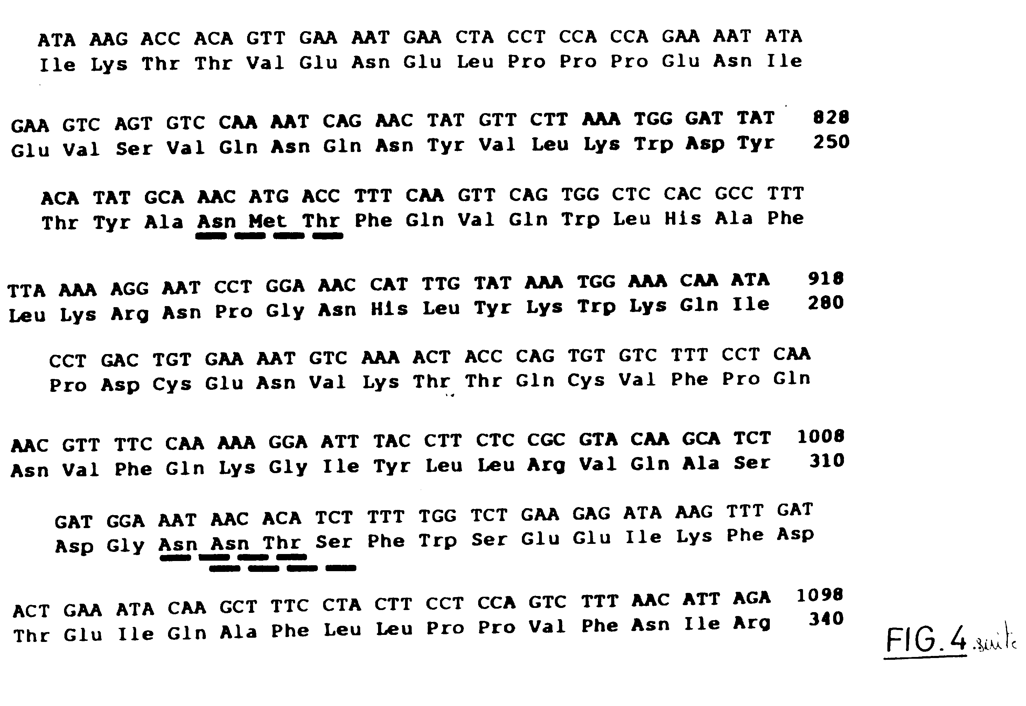

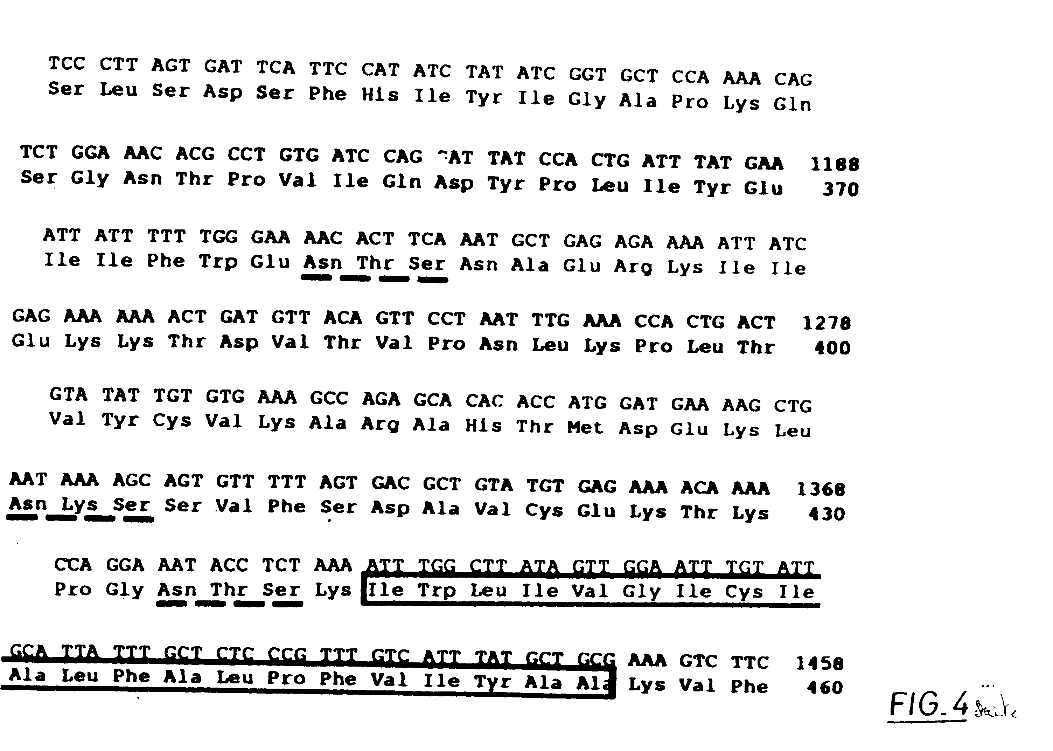

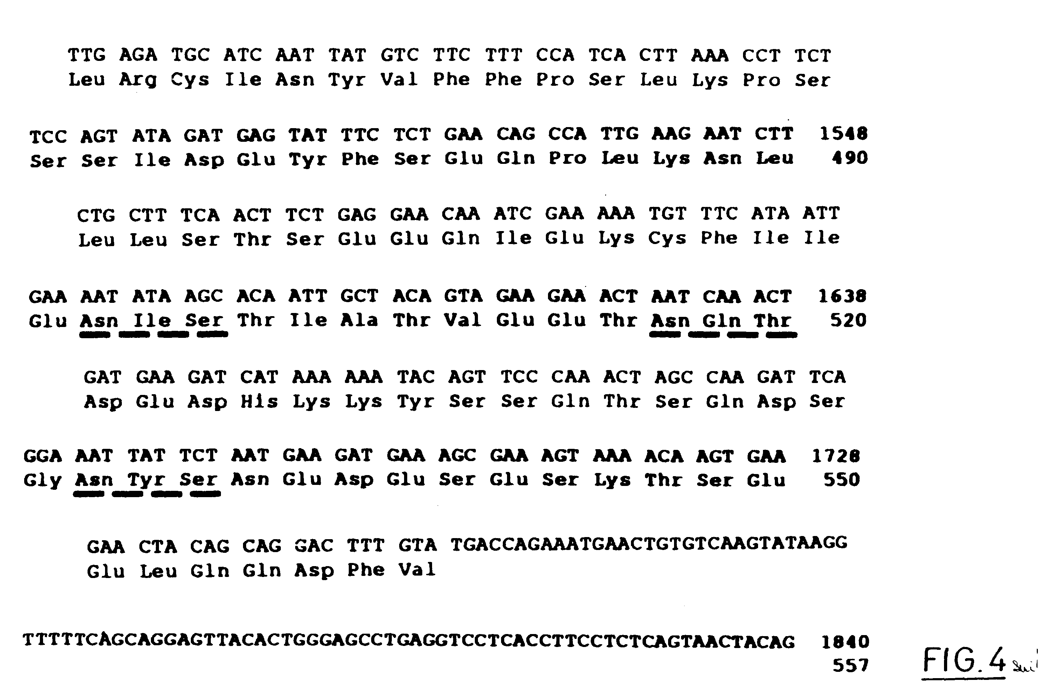

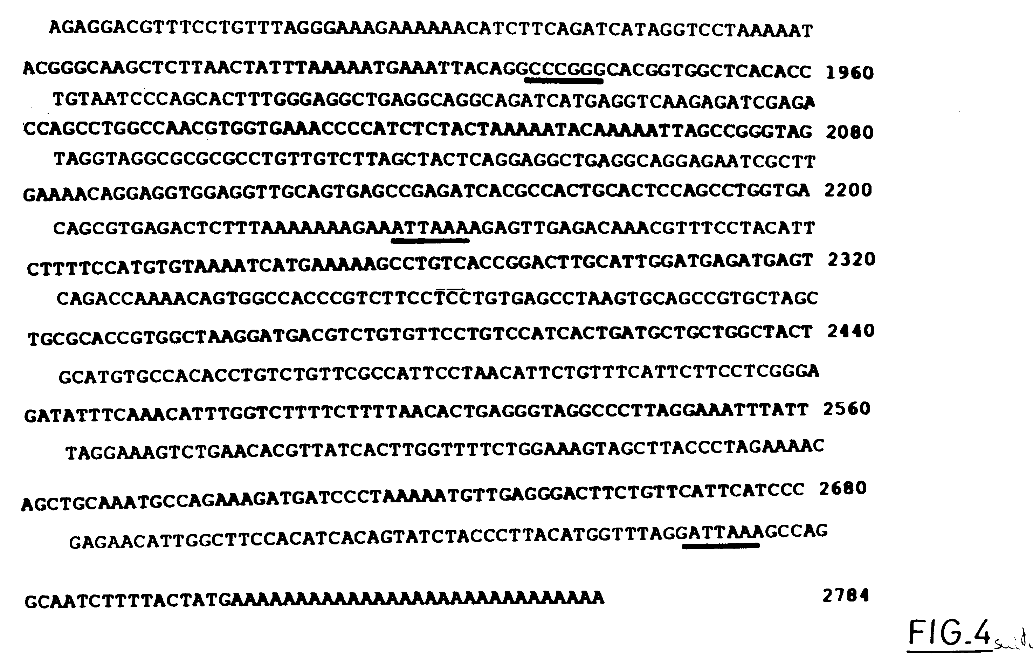

La présente invention concerne plus particulièrement l'obtention d'une protéine ayant la structure du récepteur de l'interféron alpha humain caractérisé en ce qu'il correspond à la séquence de la figure 4 ou à l'un de ses variants alléliques qui n'en diffère pas par plus de 3 acides aminés et son expression, notamment en surface de cellules ainsi que les séquences d'ADN codant pour ladite protéine.

Parmi ces variants alléliques, il peut s'agir de la séquence de la figure 4 dans laquelle la thréonine en position 164 est remplacée par une arginine et un acide aspartique est inséré entre l'acide aspartique en position 479 et l'acide glutamique en position 480.

La présente invention concerne également une séquence d'ADN codant pour le récepteur de l'interféron alpha humain.

Cette séquence d'ADN sera de préférence conforme à la séquence de la figure 4 ou à une séquence allélique de celle-ci.

La structure de la séquence d'ADN codant pour le récepteur de l'interféron alpha-humain est analysée dans les exemples. Elle comporte notamment un peptide signal. Dans certains cas, ce peptide signal pourra être supprimé ou remplacé par un autre peptide signal mais dans la plupart des cas, on préfèrera garder ce peptide signal de la figure 4 et donc la séquence codante correspondante.

Cette séquence est de préférence insérée dans un système assurant son expression cellulaire dans une cellule hôte appropriée, en particulier au niveau transmembranaire.

En particulier, la présente invention concerne le fragment d'ADN, notamment le fragment d'ADNc, caractérisé en ce qu'il code pour le gène du récepteur de l'interféron alpha conforme à la séquence de la figure 4 ou à une séquence allélique de celle-ci.

Il peut ainsi s'agir de la séquence d'ADN de la figure 4 dans laquelle la cytosine en position 569 est remplacée par une guanine et un codon TGA inséré entre l'adenine situé en position 1514 et la thymine en position 1515.

La présente invention se rapporte également aux cellules non humaines caractérisées en ce qu'elles expriment ledit récepteur de l'interféron alpha humain et à un procédé d'obtention desdites cellules.

Selon ce procécé on transfecte ou infecte des cellules hôtes compatibles avec un élément d'ADN comportant une séquence d'ADN codant pour ledit récepteur, ainsi que les éléments susceptibles d'assurer l'expression de cette séquence dans ladite cellule.

Les exemples ci-après démontrent comment cette séquence codant pour le récepteur de l'interféron alpha humain (IFN-alpha h) a pu être exprimée dans des cellules de mammifères non humains, notamment de souris. Les techniques permettant l'expression cellulaire de séquences d'ADN exogènes sont connues. Il peut s'agir suivant les cellules hôtes d'utiliser des vecteurs autoréplicatifs tels que les plasmides ou bien des vecteurs intégratifs. séquences d'ADN ou virus vecteurs par exemple. Dans le cas où l'on souhaite obtenir une lignée cellulaire exprimant le récepteur IFN-alpha humain, la technique mise en oeuvre peut être à faible rendement puisqu'on ne recherche qu'une lignée. Par contre, lorsque l'on souhaite obtenir la protéine seule, on utilisera de préférence les vecteurs assurant une amplification notamment les vecteurs plasmidiques comportant une origine de réplication ou bien les systèmes d'intégration multicopies.

La présente invention concerne en outre un procédé de préparation de récepteurs d'IFN alpha humain.

Ainsi, lorsque l'on souhaitera obtenir la protéine seule on pourra cultiver, en milieu de culture approprié, des cellules hôtes transformées, transfectées ou infectées par un vecteur d'expression de ladite protéine, comportant une séquence d'ADN codant pour ledit récepteur, sous le contrôle d'un promoteur de la transcription de cette séquence dans l'hôte ainsi que les éléments assurant la traduction de la protéine puis, séparer la protéine obtenue par tout moyen approprié.

Cette protéine pourra servir pour préparer des anticorps, notamment des anticorps monoclonaux dirigés contre le récepteur ; la technologie correspondante ne sera pas décrite en détails car il s'agit d'une technique connue.

L'invention permet donc d'obtenir :
- ledit récepteur de l'interféron alpha humain,
- les anticorps dirigés contre ce récepteur, et
- des cellules exprimant ledit récepteur.

Les applications de ces éléments sont très variées. Tout d'abord le récepteur seul ou exprimé à la surface d'une cellule peut permettre de tester les analogues de l'interféron alpha humain afin de déterminer les meilleurs agonistes.

Ce type de test peut également être envisagé grâce à la protéine correspondante fixée sur un support solide tel que plaques. billes, etc... Il s'agit de techniques qui sont déjà connues pour d'autres récepteurs ou bien pour les dosages antigène-anticorps.

Les dosages récepteurs agonistes peuvent être effectués par mesure de la fixation directe ou bien par mesure des déplacements qui permettent d'évaluer l'affinité de l'agoniste par rapport à un composé de référence, par exemple l'IFN alpha humain.

Les anticorps pourront être utilisés au dosage des récepteurs ou à leur visualisation dans le cas de l'imagerie. Il s'agit là de techniques permettant d'évaluer certains états pathologiques par exemple justifiant un traitement à l'interféron alpha ou permettant d'évaluer certains états dans lesquels le taux de ces récepteurs varie.

L'invention concerne donc également des trousses de diagnostic comprenant un ou plusieurs des éléments précédents à titre d'agent de diagnostic ou d'imagerie, ou à titre de modèle pharmacologique pour tester les produits dérivant de l'interféron alpha humain.

La protéine du récepteur ou les anticorps correspondants peuvent également être utilisés à titre d'agent pharmacologique lorsque l'on souhaitera bloquer les récepteur d'IFN alpha humain ou bien lorsque l'on utilisera la protéine à titre de leurre pour bloquer l'IFN alpha humain dans certains états où l'hyperexpression de l'interféron alpha humain peut être nuisible.

Enfin, les anticorps pourront être utilisés comme agent de pilotage pour insérer un principe actif couplé à cet anticorps au voisinage d'un récepteur de l'IFN alpha humain.

Les exemples et figures donnés ci-dessous à titre non limitatif permettront de mettre en évidence d'autres avantages et caractéristiques de la présente invention.

La figure 1 représente les courbes de liaison de l'interféron humain alpha B (symboles noirs) ou de l'hybride BDBB (symboles blancs) sur le transfectant primaire 10BH7 (A) ou sur les cellules parentales BTG 9A (B).

Les figures 2 représentent l'analyse en "Southern blot" de l'ADN génomique des transfectants primaires et secondaires.
Figure 2A :
   Puit n°1 : Digestion EcoRI de l'ADN du clone primaire 10BH7 hybridé avec une sonde Alu.
   Puit n° 2 : même ADN hybridé avec une sonde Lambda.
Figure 2B:
   Puits n° 1 et 3 : Digestion BamHI de l'ADN des deux transfectants secondaires 1B4D10 et 2A415 hybridé avec une sonde Alu.
   Puits n° 2 et 4 : Digestion BamHI de l'ADN de deux transfectants secondaires négatifs hybridé avec une sonde Alu.
   Puit n° 5 : Digestion BamHI de l'ADN des cellules parentales BTG 9A hybridé avec une sonde Alu.

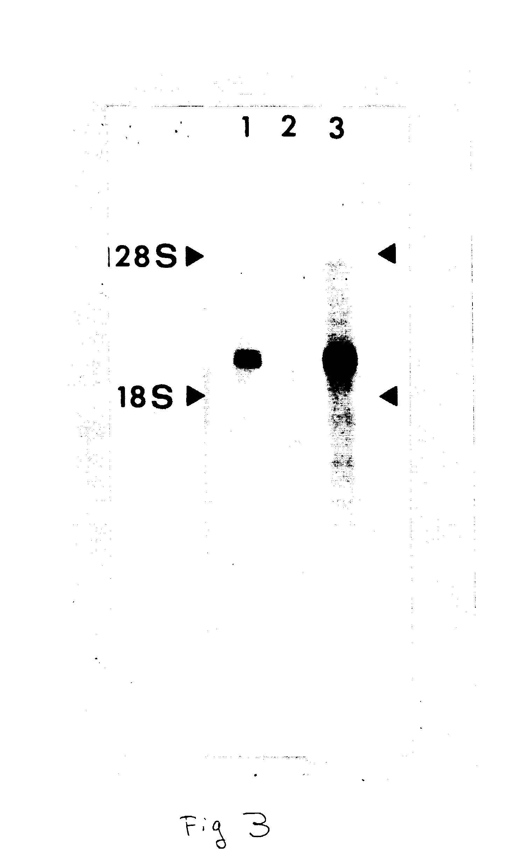

La figure 3 représente l'analyse en "northern blot" des ARN polyA+ des deux transfectants secondaires (puits n° 1 et 3) et des cellules parentales (puits n°2), hybridé avec la sonde Eco RI 5 kb.

La figure 4 représente la séquence nucléotidique de l'ADNc du récepteur de l'interféron humain alpha ainsi que sa séquence en acide aminé. Le peptide signal et la région transmembranaire sont encadrés. Les sites de glycosilation liés à l'azote sont soulignés par des tirets. Les deux sites de polyadénylation et le site de restriction Sma I sont soulignés.

### EXEMPLE 1 :

### Sélection de cellules de souris BTG transfectées sensibles à l'interféron alpha B humain.

Des cellules BTG 9A de souris sont co-transfectées avec une banque d'ADNc humain clonée dans un vecteur d'expression phage lambda de mammifères contenant également le gène neo bactérien et de l'ADN génomique Daudi de haut poids moléculaire, dans un rapport 1 : 1. Ce système de co-transfection où l'expression d'un récepteur interféron humain peut provenir de l'ADN humain génomique et/ou de l'ADNc humain est utilisé pour augmenter les chances d'isoler des transfectants intéressants.

Des clones de transfectants stables sont sélectionnés dans un milieu G418 avec une fréquence de 10⁻² - 10⁻³ . Afin de détecter les clones cellulaires sensibles à l'interféron alpha humain, des clones transfectés sont traités avec 30 000 unités/ml d'interféron alpha B humain et infectés avec du VSV. A cette concentration, des cellules BTG de souris sont insensibles à l'interféron alpha B, mais un clone transfectant exprimant le gène récepteur de l'interféron alpha humain devrait être à un stade antiviral.

Compte tenu du fait que le titre interféron est inversement proportionnel à la multiplicité de l'infection, cette sélection virale permet de neutraliser l'importante quantité de VSV produite par la majorité des clones des cellules inappropriées qui aboliraient l'état antiviral du clone de la cellule intéressante. Cette méthode implique une rapide adsorption du virus sur les cellules suivie d' un traitement avec de l'antisérum anti-VSV de lapin pour neutraliser l'excès du virus, et un développement de l'effet cytopathique dans un antisérum anti-VSV de lapin contenant un milieu de gélose semi-solide. Des clones cellulaires survivants sont individuellement isolés. Pour éviter une infection VSV chronique, les clones de cellules sont soumis à un traitement avec un interféron alpha/béta de souris et l' antisérum anti-VSV est maintenu dans un milieu G-418 durant une semaine. Ils sont ensuite retestés pour leur sensibilité à l'interféron alpha B humain vis-à-vis des infections VSV ou EMC. La plupart de ces clones transfectés sont insensibles à l'interféron alpha B humain. Néanmoins, un clone révéle une sensibilité intéressante à l'interféron alpha B humain. Il est alors sous cloné et appelé 10B H7.

On détermine la sensibilité des cellules 10B H7 à de nombreux interférons alpha humains et murins puis on compare le comportement de ces cellules avec les cellules BTG murines parentales.

Le tableau I montre l'activité de l'interféron alpha/béta de souris, l'interféron alpha B humain, l'interféron-béta humain et l'interféron gamma humain testés sur des cellules BTG de souris parentales. du clone 10B H7 transfecté et des cellules humaines Wish en utilisant en tant que virus le VSV ou l'EMC.

En ce qui concerne l'interféron murin, les cellules 10B H7 sont aussi sensibles que les cellules BTG parentales. D'autre part, les cellules 10B H7 manifestent au moins une sensibilité 64 000 fois plus importante à l'interféron alpha B humain comparées aux cellules parentales. On observe également une augmentation 8 fois plus importante de l'activité de l'interféron béta humain sur les cellules 10B H7 mais on ne détecte aucune activité antivirale de l'interféron gamma humain qui est reconnu par un récepteur distinct aussi bien sur les cellules BTG parentales que sur le clone 10B H7 transfecté. L'activité antivirale spécifique de l'interféron alpha B (4.7. 10⁶ unités/mg) sur les cellules 10B H7 est de l'ordre des activités spécifiques des interférons alpha humains sur les cellules humaines.

**TABLEAU I**

| **Activités antivirales de préparations d'interféron testées sur : (Unités/ml)** | | | | |
|---|---|---|---|---|
| | **BTG** | **10BH7** | **WISH** | **RAPPORT 10BH7/BTG** |
| IFN alpha/béta de souris | 12,8x10⁶ | 12,8x10⁶ | 240 | 1 |
| IFN alphaB humain | <20 | 1,27x10⁶ | 54X10⁶ | > 64 000 |
| IFN béta humain | 200 | 1,6x10³ | 2,7x10⁶ | 8 |
| IFN gamma humain | <1x10³ | <1x10³ | 20x10⁶ | --- |

### EXEMPLE 2 :

### Transfectants d'une cellule BTG de souris sensibles à l'interféron exprimant le récepteur d'interféron humain.

Sur des cellules 10B H7, l'interféron alpha B humain se comporte comme un interféron alpha D sur des cellules humaines, activité spécifique similaire et affinité de liaison apparente similaire. Comme l'interféron alpha D sur des cellules humaines, la liaison de l'interféron alpha B humain sur des cellules 10B H7 peut être mesurée seulement à 37°C.

On réalise plusieurs expériences de liaison avec de l'interféron alpha B iodé utilisé en tant que sonde pour le récepteur humain et un interféron hybride iodé appelé BDBB qui est actif à la fois sur les lignées de souris parentales et sur le clone 10B H7, en tant que contrôle positif. Cet interféron hybride qui a une activité spécifique sur les cellules de souris, proche de celle de l'interféron alpha B humain sur les cellules 10B H7, pourrait être une sonde pour les récepteurs humains et de souris à la fois sur les cellules BTG parentales et les cellules 10B H7 transfectées.

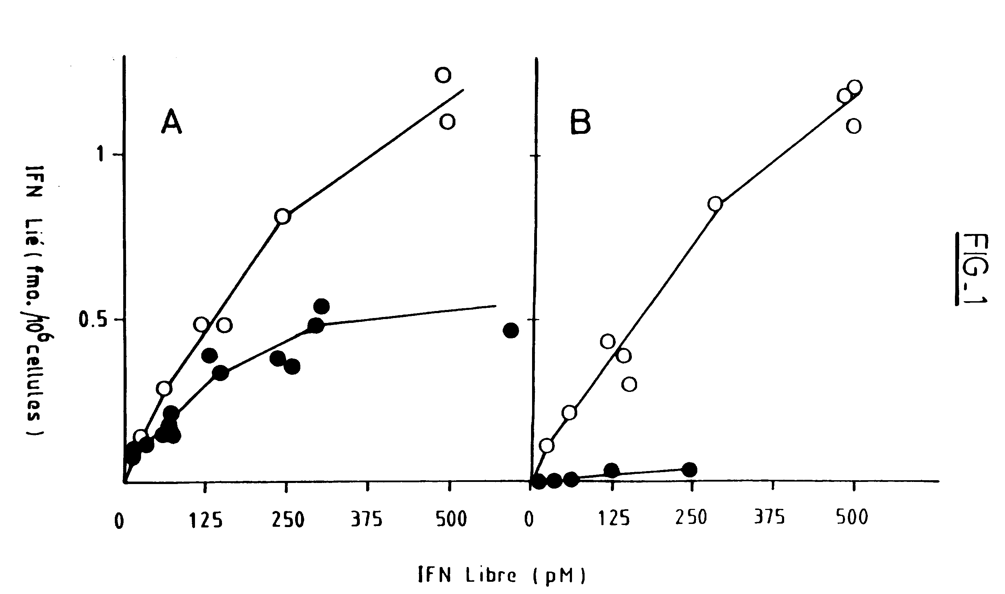

La figure 1 montre que la liaison du BDBB est similaire sur des cellules BTG et 10B H7. Au contraire, les cellules 10B H7, se lient spécifiquement à l'interféron alpha B alors que les cellules BTG ne le font pas. Les paramètres de liaison calculés d'après les données de Scatchard montrent que les cellules 10B H7 expriment environ 500 sites de liaison par cellule pour l'interféron alpha B avec un Kd apparent de 2.10⁻¹⁰ M. Ceci est proche des valeurs de l'interféron BDBB (1 500 sites de liaison par cellule ; KD apparent 5.10⁻¹⁰ M), qui est actif à la fois sur les lignées de souris parentales et sur le clone 10B H7.

En conclusion, ces résultats complétés avec d'autres études. indiquent que l'interféron alpha B humain se lie avec un récepteur spécifique sur les cellules 10B H7 et non pas sur les cellules des souris parentales.

### EXEMPLE 3 :

### Clonage d'une sonde couvrant le gène du récepteur interféron alpha humain dans des clones d'une cellule transfectée secondaire

Partant de l'hypothèse que les cellules 10B H7 expriment un gène humain transfecté nécessaire pour conférer des sites de liaison et une activité antivirale de l'interféron alpha B sur une cellule de souris, il a été recherché la distribution de l'ADN humain dans le génome 10B H7 transfecté.

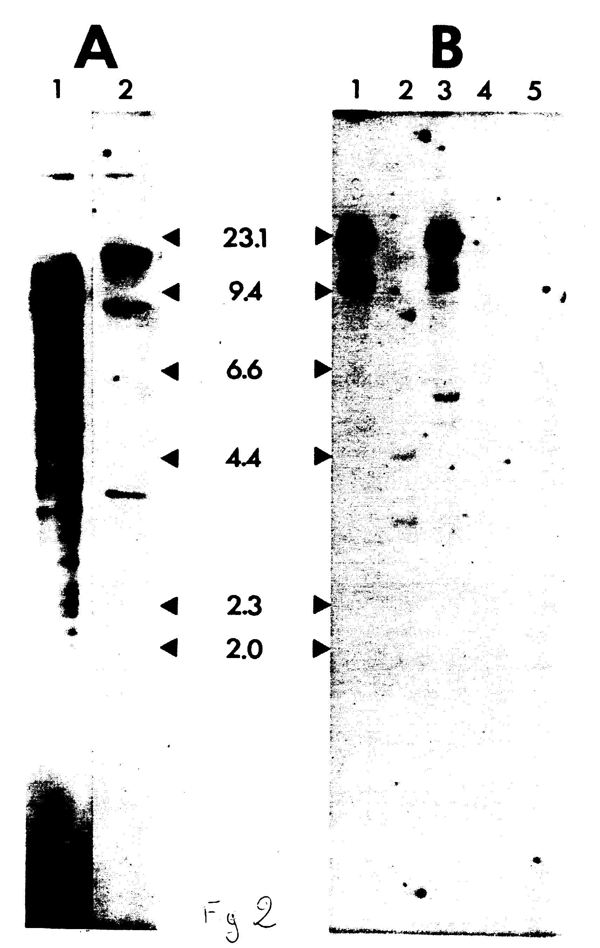

La figure 2A est un "southern blot" avec l'ADN du clone 10B H7 utiiisant soit une séquence Alu humaine détectant l'ADN génomique humain soit une sonde à ADN lambda pour détecter l'ADNc intégré.

On a ainsi montré que les transfectants avaient intégré plus qu'une part de 1000 de l'ADN Daudi humain et 100 copies de l'ADNc humain de la banque d'expression des mammifères. En raison de l'importante quantité d'ADN humain dans ce clone, il a été nécessaire d'isoler des transfectants secondaires afin de cloner la séquence d'ADN responsable de l'expression du récepteur dans le clone 10B H7 initial. Dans ce but, on a co-transfecté des cellules de souris BTG avec de l'ADN génomique du clone 10B H7 et du neopSV2. Les cellules sensibles à l'interféron humain ont été isolées. Les transfectants secondaires stables sont soumis à 4 cycles de traitement de l'interféron alpha B et à l'infection VSV. Après sous clonage, il a été obtenu deux clones secondaires indépendants 1B4D10 et 2A4l5. Ces deux clones secondaires sont indépendants mais dérivent du même clone initial. Ils ont les mêmes caractéristiques que celles du clone initial 10B H7, c'est-à-dire sensibilité à l'interféron alpha humain et récepteur d'expression à leur surface. Ces deux clones secondaires ne retiennent pas les séquences associées aux phages lambda dans leur génome et en conséquence l'expression des récepteurs de l'interféron alpha humain est dû à l'ADN.
génomique des cellules Daudi qui avait été transfecté initialement dans les cellules BTG. Elles ont en effet conservé les séquences génomiques humaines. La figure 2B est une digestion de BamHI des clones d'ADN secondaires hybridés avec une sonde ALU qui détecte les séquences répétititives ALU. Les deux clones secondaires positifs 1B4D10 et 2A415 ont en commun une bande principale de 18 kb.

Une sonde ALU a été utilisée pour cribler une banque génomique de taille fractionnée (20-15 kb) de la digestion complète de BamHI, de l'ADN du clone secondaire 1B4D10 cloné dans un phage lambda EMBL 3. Les séquences répétées ALU contenant le fragment BamHI de 18 kb ont été isolées puis sous clonées dans un vecteur plasmide pUC.

Ce fragment contient deux sites EcoRI donnant des fragments d'extrémité de Il kb et de 2 kb et un fragment central de 5 kb. Il y a des séquences répétitives ALU humaine dans ces trois fragments et donc le fragment 5 kb central doit être débarrassé des séquences ADN de la souris. Le fragment Eco RI 5 kb central est présent dans une digestion Eco RI de l'ADN des deux clones secondaires 1B4D10 et 2A4 15, obtenus indépendamment du même clone initial.

### EXEMPLE 4 :

### Clonage et séquence nucléotidique de l' ADNc du récepteur de l'interféron alpha humain

La figure 3 montre que la sonde EcoRI 5kb détecte dans les clones secondaires un transcrit de taille égale, absent de l'ARN poly A⁺ des cellules BTG parentales.

On crible une banque d'ADNc préparée dans un vecteur phage ZapII lambda à partir de l'ARN du clone secondaire 1B4 D10 et on isole l'ADNc hybridant la sonde EcoRI 5 kb. Huit clones d'ADNc indépendants, tous portant la même extrémité 3' sont analysés par séquençage. Les plus longs (1 900 bp) ont à leur extrémité 5' un fragment de restriction HindIII de 400 bp couvrant seulement les séquences de codage et sont dépourvus d'éléments de répétition. Ce fragment utilisé en tant que sonde détecte le transcript 2,5 kbp présent à la fois dans les clones secondaires et dans les cellules humaines Daudi mais absent des cellules BTG de la souris. Vu que des points de mutation dans des gènes transfectés peuvent se présenter, cette sonde HindIII de 400 bp a été utilisée pour étudier une banque d'ADNc ZapII lambda à partir de cellules humaines Daudi afin d'isoler des clones d'ADNc complets correspondants au transcript humain.

Des chevauchements d'ADNc isolés à partir de banques d'ADNc Daudi humain ont été préservés dans des plasmides "pBluescript" à partir de l'excision in vivo de Lambda ZapII par des phages assistants f1. L'ADN à simple brin récupéré en présence du phage intermédiaire M 13 à partir des bactéries contenant des plasmides "pBluescript" est séquencé sur une extrémité de l'ADNc par la méthode de terminaison de chaîne (Sanger et al. 1977) et les séquences de l'autre extrémité de l'ADNc sont obtenues à partir de la séquence à double brin sur plasmide. Des oligonucléotides ont été synthétisés et utilisés notamment en séquençage de l'ADN quand des "gaps" apparaissaient dans la séquence.

Les deux brins de l'ADNc les plus longs décrits en figure 4 ont été complètement séquences.

La séquence d'ADNc est de l'ordre de 2784 bp et contient une région non traduite de 1035 bp 3', qui inclut deux séquences de polyadénylation ATTAAA. La séquence du cadre de lecture ouvert est complète car elle est terminée en 5' par un codon STOP. Deux codons ATG sont trouvés côte à côte en position 79 et 82.

En plus de la région hydrophobique de l'amino terminal, on identifie une seconde région hydrophobique (acide aminé 456 à 476). Il existe 15 sites de glycosilation liés à l'azote potentiels, 12 dans le domaine extracellulaire putatif et 3 dans le domaine intracellulaire putatif.

Le poids moléculaire de la séquence proposée comme récepteur (le peptide signal inclus) est de 63 485 Dalton, la glycosilation pouvant rendre compte de l'ordre de 95 000-100 000 Dalton pour la protéine du récepteur naturel.

Le récepteur de l'interféron alpha humain apparaît être une protéine unique. Sa séquence présente néanmoins une certaine variation allélique. Une telle variation est par exemple repérée dans les cellules hétérozygotes Daudi, qui exprime deux allèles du récepteur, l'un étant conforme à la séquence décrite dans la figure 4, l'autre présentant une substitution d'une cytosine par une guanine en position 569 et une insertion de trois bases T, G et A après l'adénine située en position 1514. Au niveau protéique, il en résulte la substitution de la thréonine 164 par une arginine, et par l'insertion d'un acide aspartique après l'acide aspartique 479.

## Revendications

1. Séquence d'ADN caractérisée en ce qu'elle code pour une protéine de récepteur correspondant à la séquence de la figure 4 ou à l'un de ses variants alléliques qui n'en diffère pas par plus de 3 amino acides.

2. Séquence d'ADN selon la revendication 1, caractérisée en ce qu'il s'agit de la séquence d'ADN de la figure 4.

3. Séquence d'ADN selon la revendication 1, caratérisée en ce qu'il s'agit de la séquence d'ADN de la figure 4 dans laquelle la cytosine en position 569 est remplacée par une guanine, et en ce qu'un codon TGA est inséré entre l'adénine située en position 1514 et la thymine située en position 1515.

4. Protéine résultant de l'expression dans une cellule hôte d'une séquence d'ADN selon l'une quelconque des revendications 1 à 3.

5. Récepteur de l'interféron alpha humain caractérisé en ce qu'il correspond à la séquence de la figure 4 ou à l'un de ses variants alléliques qui n'en diffère pas par plus de 3 amino acides.

6. Récepteur de l'interféron alpha humain selon la revendication 5, caractérisé en ce qu'il comporte en outre un peptide signal.

7. Récepteur de l'interféron alpha humain selon la revendication 6, caractérisé en ce que le peptide signal correspond au peptide signal de la figure 4.

8. Récepteur de l'interféron alpha humain selon l'une quelconque des revendications 5 à 7, caractérisé en ce qu'il correspond à la séquence de la figure 4.

9. Récepteur de l'interféron alpha humain selon l'une des revendications 5 à 7, caractérisé en ce qu'il correspond à la séquence de la figure 4 dans laquelle la thréonine en position 164 est remplacée par une arginine, et en ce qu'un acide aspartique est inséré entre l'acide aspartique en position 479 et l'acide glutamique en position 480.

10. Cellule non humaine caractérisée en ce qu'elle exprime un récepteur selon l'une des revendications 5 à 9.

11. Cellule selon la revendication 10, caractérisée en ce qu'il s'agit d'une cellule de mammifère.

12. Cellule selon la revendication 11, caractérisée en ce qu'il s'agit d'une cellule de souris.

13. Procédé d'obtention de cellules exprimant le récepteur selon l'une des revendications 5 à 9, caractérisé en ce qu'on transforme, transfecte ou infecte des cellules hôtes compatibles avec un élément d'ADN comportant une séquence d'ADN selon l'une des revendications 1 à 3, ainsi que les éléments susceptibles d'assurer l'expression de cette séquence dans lesdites cellules.

14. Procédé de préparation d'une récepteur d'lFN alpha humain caractérisé en ce qu'on cultive sur un milieu de culture approprié des cellules hôtes transformées, transfectées ou infectées par un vecteur d'expression de ladite protéine comportant une séquence d'ADN selon l'une des revendications 1 à 3 sous le contrôle d'un promoteur de la transcription de cette séquence dans l'hôte ainsi que des éléments assurant la traduction de la protéine et en ce que l'on sépare ladite protéine après culture.

15. Anticorps dressés contre les récepteurs selon l'une des revendications 5 à 9.

16. Kit de diagnostic, caractérisé en ce qu'il comprend au moins un anticorps selon la revendication 15 ou un récepteur selon l'une des revendications 5 à 9.

17. Application des récepteurs selon l'une des revendications 5 à 9 ou de cellules selon l'une des revendications 10 à 12 à la mise en évidence d'agonistes de l'interféron alpha humain par mesure des affinités de ces agonistes par rapport à l'IFN alpha humain.

## Claims

1. DNA sequence characterized in that it codes for a receptor protein corresponding to the sequence of figure 4 or to one of its allelic variants which does not differ from it by more than 3 amino acids.

2. DNA sequence according to Claim 1, characterized in that it is the DNA sequence of figure 4.

3. DNA sequence according to Claim 1, characterized in that it is the DNA sequence of figure 4 in which the cytosine at position 569 is replaced by a guanine, and in that a TGA codon is inserted between the adenine situated at position 1514 and the thymine situated at position 1515.

4. Protéine resulting from the expression in a host cell of a DNA sequence according to any one of Claims 1 to 3.

5. Receptor for human alpha interferon characterized in that it corresponds to the sequence of figure 4 or to one of its allelic variants which does not differ from it by more than 3 amino acids.

6. Receptor for human alpha interferon according to Claim 5, characterized in that it bears in addition a signal peptide.

7. Receptor for human alpha interferon according to Claim 6, characterized in that the signal peptide corresponds to the signal peptide of figure 4.

8. Receptor for human alpha interferon according to one of the Claims 5 to 7, characterized in that it corresponds to the sequence of figure 4.

9. Receptor for human alpha interferon according to one of the Claims 5 to 7, characterized in that it corresponds to the sequence of figure 4 in which the threonine at position 164 is replaced by an arginine, and in that an aspartic acid is inserted between the aspartic acid at position 479 and the glutamic acid at position 480.

10. Non-human cell characterized in that it expresses a receptor according to one of the Claims 5 to 9.

11. Cell according to Claim 10, characterized in that it is a mammalian cell.

12. Cell according to Claim 11, characterized in that it is a mouse cell.

13. Process for the production of cells expressing the receptor according to one of the Claims 5 to 9, characterized in that compatible host cells are transformed, transfected or infected with an element of DNA bearing a DNA sequence according to one of the Claims 1 to 3 as well as the elements capable of ensuring the expression of this sequence in the said cells.

14. Process for the preparation of a human alpha IFN receptor characterized in that host cells transformed, transfected or infected by an expression vector of the said protein bearing a DNA sequence according to one of the Claims 1 to 3 under the control of a promoter of the transcription of this sequence in the host as well as elements ensuring the translation of the protein, are cultivated on a suitable culture medium and in that the said protein is separated after culture.

15. Antibodies directed against the receptors according to one of the Claims 5 to 9.

16. Diagnostic kit, characterized in that it comprises at least one antibody according to Claim 15, or one receptor according to one of the Claims 5 to 9.

17. Application of the receptors according to one of the Claims 5 to 9 or cells according to one of the Claims 10 to 12 for the detection of agonists of human alpha interferon by measurement of the affinities of these agonists compared with human alpha IFN.

## Patentansprüche

1. DNS-Sequenz, dadurch **gekennzeichnet**, dass sie für ein Rezeptorprotein codiert, welches der Sequenz der Fig. 4 oder einer ihrer allelischen Varianten entspricht, die sich in nicht mehr als drei Aminosäuren von dieser unterscheiden.

2. DNS-Sequenz gemäss Anspruch 1, dadurch **gekennzeichnet**, dass es sich um die DNS-Sequenz der Fig. 4 handelt.

3. DNS-Sequenz gemäss Anspruch 1, dadurch **gekennzeichnet**, dass es sich um die DNS-Sequenz der Fig. 4 handelt, in der das Cytosin in der Position 569 durch ein Guanin ersetzt ist, und dadurch, dass ein TGA-Codon zwischen dem Adenin in der Position 1514 und dem Thymin in der Position 1515 eingefügt ist.

4. Protein, welches aus der Expression in einer Wirtszelle einer DNS-Sequenz gemäss einem der Ansprüche 1 bis 3 resultiert.

5. Human-Interferon-α-Rezeptor, dadurch **gekennzeichnet**, dass er der Sequenz der Fig. 4 oder einer ihrer allelischen Varianten entspricht, die sich in nicht mehr als drei Aminosäuren von dieser unterscheidet.

6. Human-Interferon-α-Rezeptor gemäss Anspruch 5, dadurch **gekennzeichnet**, dass er ausserdem ein Signalpeptid trägt.

7. Human-Interferon-α-Rezeptor gemäss Anspruch 6, dadurch **gekennzeichnet**, dass das Signalpeptid dem Signalpeptid der Fig. 4 entspricht.

8. Human-Interferon-α-Rezeptor gemäss einem der Ansprüche 5 bis 7, dadurch **gekennzeichnet**, dass er der Sequenz der Fig. 4 entspricht.

9. Human-Interferon-α-Rezeptor gemäss einem der Ansprüche 5 bis 7, dadurch **gekennzeichnet**, dass er der Sequenz der Fig. 4 entspricht, in der das Threonin in der Position 164 durch ein Arginin ersetzt wurde, und dadurch, dass eine Asparaginsäure zwischen der Asparaginsäure in der Position 479-und der Glutaminsäure in Position 480 eingefügt wurde.

10. Nicht-humane Zelle, dadurch **gekennzeichnet**, dass sie einen Rezeptor gemäss einem der Ansprüche 5 bis 9 exprimiert.

11. Zelle gemäss Anspruch 10, dadurch **gekennzeichnet**, dass es sich um eine Mammiferenzelle handelt.

12. Zelle gemäss Anspruch 11, dadurch **gekennzeichnet**, dass es sich um eine Mäusezelle handelt.

13. Verfahren zum Erhalt der Zellen, die den Rezeptor gemäss einem der Ansprüche 5 bis 9 exprimieren, dadurch **gekennzeichnet**, dass man kompatible Wirtszellen mit einem DNS-Element, das eine DNS-Sequenz gemäss einem der Ansprüche 1 bis 3 trägt, sowie geeigneten Elementen, die die Expression dieser Sequenz in diesen Zellen sicherstellen, transformiert, transfiziert oder infiziert.

14. Verfahren zur Herstellung eines Human-IFN-α-Rezeptors, dadurch **gekennzeichnet**, dass man auf einem geeigneten Kulturmedium Wirtszellen kultiviert, die mit einem Expressionsvektor des besagten Proteins, welcher eine DNS-Sequenz gemäss einem der Ansprüche 1 bis 3 unter Kontrolle eines Transcriptionspromotors dieser Sequenz in dem Wirt sowie Elemente trägt, die die Transduktion des Proteins sicherstellen, transformiert, transferiert oder infiziert wurden, und dass man das besagte Protein hiervon nach der Kultivierung abtrennt.

15. Antikörper, die sich gegen den Rezeptor gemäss einem der Ansprüche 5 bis 9 richten.

16. Diagnostischer Kit, dadurch **gekennzeichnet**, dass er mindestens einen Antikörper gemäss Anspruch 15 und einen Rezeptor gemäss einem der Ansprüche 5 bis 9 umfasst.

17. Verwendung des Rezeptors gemäss einem der Ansprüche 5 bis 9 oder der Zellen gemäss einem der Ansprüche 10 bis 12 zum Nachweis von Human-Interferon-α-Agonisten durch Messung der Affinität dieser Agonisten in bezug auf das Human-IFN-α.
